# EUROPÄISCHE PATENTSCHRIFT

(11) **EP 3 112 448 B1**
(45) Veröffentlichungstag und Bekanntmachung des Hinweises auf die Patenterteilung: **21.03.2018**
(21) Anmeldenummer: 15174508.0
(22) Anmeldetag: 30.06.2015
(51) Int. Cl.: C11B 9/00, C07C 69/24, C07C 69/68

(54) **DERIVATE DES 1-(4-METHYLCYCLOHEXYL)-ETHANOLS**
DERIVATIVES OF 1- (4-METHYLCYCLOHEXYL) ETHANOL
DERIVES D'ETHANOLS 1-(4-METHYLCYCLOHEXYL)

(43) Veröffentlichungstag der Anmeldung: 04.01.2017
(73) Patentinhaber: Symrise AG, 37603 Holzminden (DE)
(72) Erfinder: Dilk, Erich, 37603 Holzminden (DE); Diaz, Edison, 38642 Goslar (DE); Kurzenne, Pierre, 92270 Bois Colombes (FR); Kuhn, Walter, 37603 Holzminden (DE)
(74) Vertreter: Copsey, Timothy Graham

(56) Entgegenhaltungen:
- EP-A1- 2 281 581
- DE-A1- 2 650 602

## Beschreibung

### Gebiet der Erfindung

Die vorliegende Erfindung betrifft das Gebiet der Duftstoffe.

### Stand der Technik

DE2650602 A1 offenbart einen Strukturraum, der Derivate des 1-(4-Methylcyclohexyl)-ethanols neben Derivaten vieler weiterer Verbindungen umfasst, wobei Verbindungen innerhalb des Strukturraumes gegen unangenehme Gerüche (Desodorierungsmittel) verwendet werden. Die Verbindungen lindern die widerwärtige Wirkung eines übelriechenden Stoffs oder eines üblen Geruchs auf den menschlichen Geruchssinn, ohne dass sich diese Wirkung auf einen besonderen Mechanismus bezieht. Für die explizit genannten Derivate des 1-(4-Methylcyclohexyl)-ethanols aus den Beispielen 25 und 26 wird keinerlei Geruch angegeben. Außerdem wird in den Beispielen gezeigt, dass die Verbindungen 25 und 26 den Geruch von Schlechtgerüchen vermindern können, bis hin zu einem reinen Geruch.

### Aufgabe der Erfindung

Es ist eine Aufgabe der Erfindung, Mittel zur Verstärkung von Wohlgerüchen bereitzustellen.

### Beschreibung der Erfindung

Die Aufgabe wird gelöst durch eine Mischung, enthaltend
mindestens einen Duftstoff der Formel (I) 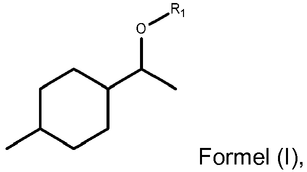 wobei
R1 = OC-R2, CH2OR3, C1-C8-offenkettiger oder verzweigten aliphatischen Rest, gegebenenfalls substituiert und/oder ungesättigt,
mit R2 = einen offenkettigen oder verzweigten aliphatischen Rest, gegebenenfalls substituiert und/oder ungesättigt, mit 2-10 C-Atomen,
mit R3 = einen offenkettigen oder verzweigten aliphatischen Rest, gegebenenfalls substituiert und/oder ungesättigt, mit 1-8 C-Atomen bedeuten und
b) mindestens einen Duftstoff, verschieden von den Duftstoffen der Komponente a, dadurch gekennzeichnet, dass das Gewichtsverhältnis aller Komponenten a zu allen Komponenten b von 1:10 bis 1:10000 beträgt.

Anhand des zitierten Standes der Technik ist es überraschend, dass diese Mischungen Wohlgerüche verstärken können, da die Verbindungen des allgemeinen Strukturraums aus DE2650602 A1 verwendet werden, um Gerüche zu vermindern, also genau das Gegenteil der erfindungsgemäßen Aufgabe bewirken.

Die Verbindungen der Formel (I) können insbesondere als Rest R1 einen Propanoyl-, Butanoyl-, 2-Methylpropanoyl-, 2-Hydroxypropanoyl-, Methoxyethyl- oder 2-Methylbut-2-enyl-Rest enthalten.

Die Mischung kann mindestens zwei Duftstoffe der Formel (I) enthalten.

Insbesondere kann es sich bei den Verbindungen der Formel (I) um eine oder mehrere der nachfolgend in der Tabelle 1 aufgelisteten Verbindungen handeln.

Die in Tabelle 1 genannten Duftstoffe selbst besitzen überwiegend blumige und fruchtige Geruchseigenschaften und können daher auch selbst als Duftstoffe, insbesondere zum Erzeugen blumiger und fruchtiger Geruchsnoten verwendet werden. Neben ihrem Eigengeruch können die Duftstoffe Wohlgerüche verstärken, wobei insbesondere die Wohlgerüche blumig oder/ und fruchtig verstärkt werden. Die Verstärkung der Wohlgerüche, insbesondere der blumigen oder/ und fruchtigen Wohlgerüche, kann in einer synergistischen Weise erfolgen.

Einige Verbindungen der Formel (I) sind neu, es wird angenommen, dass die Verbindungen 1, 4, 5 und 6 aus Tabelle 1 neu sind.

**Tabelle 1**

| | Formel | Name | Geruchliche Beschreibung |
|---|---|---|---|
| 1 | 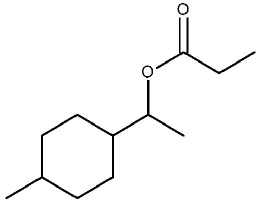 | 1-(4-Methylcyclohexyl)ethyl-propionat | Rose, |
| | | | Geranyl |
| | | | Neryl |
| | | | Litschi |
| 2 | 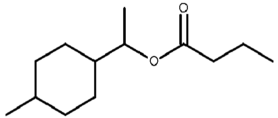 | 1-(4-Methylcyclohexyl)ethyl-butyrat | Rose |
| | | | Blüte |
| | | | Carbinol |
| | | | Pfirsich |
| 3 | 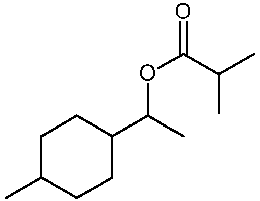 | 1-(4-Methylcyclohexyl)ethyl-isobutyrat | Rose |
| | | | Blüte |
| 4 | 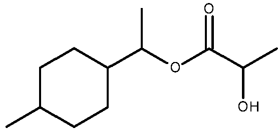 | 1-(4-Methylcyclohexyl)ethyl-2-hydroxypropionat | Veilchen |
| | | | Lilie |
| | | | Grün |
| 5 | 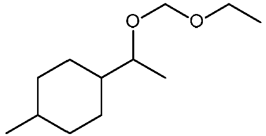 | 1-[1-Ethoxymethoxy]ethyl]-4-methylcyclohexan | Grüner Apfel |
| | | | Maiglöckchen |
| 6 | 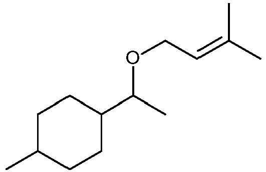 | 1-Methyl-4-[1-(3-Methylbut-2-enoxy)-ethyl]-cyclohexan | blumig, fruchtig |

Beispiele für den weiteren mindestens einen Duftstoff (Komponente b) finden sich z.B. in S. Arctander, Perfume and Flavor Materials, Vol. I und II, Montclair, N. J., 1969, Selbstverlag oder H. Surburg und J. Panten, Common Fragrance and Flavor Materials, 5th Ed., Wiley-VCH, Weinheim 2006.

Der weitere mindestens eine Duftstoff (Komponente b) kann beispielsweise sein:
Ein oder mehrere Extrakte aus natürlichen Rohstoffen, wie Etherische Öle, Concretes, Absolues, Resine, Resinoide, Balsame, Tinkturen wie z. B.

Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreumabsolue; Cedernblätteröl; Cedernholzöl; Cistusöl; Citronellöl; Citronenöl; Copaivabalsam ; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptuscitriodora-ÖI; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-ÖI; Lorbeerblätteröl; Macisöl; Majoranöl; Mandarinenöl; Massoirindenöl; Mimosa-Absolue; Moschuskörneröl; Moschustinktur; Muskateller-Salbei-ÖI; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-ÖI; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl, sowie Fraktionen davon, bzw. daraus isolierten Inhaltsstoffen;

Einzel-Riechstoffe oder/und Mischungen aus den folgenden Gruppen:
Gruppe der Kohlenwasserstoffe, wie z. B. 3-Caren; α- Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z. B.
Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z. B.
Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;
der aliphatischen Ketone und deren Oxime wie z. B.
2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; 6-Methyl-5-hepten-2-on;
der aliphatischen schwefelhaltigen Verbindungen wie z. B.; 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B.; 2-Nonensäurenitril; 2-Tridecensäurenitril; 2,12-Tridecadiensäurenitril; 3,7-Dimethyl-2,6-octadiensäurenitril; 3,7-Dimethyl-6-octensäurenitril; der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate; der acyclischen Terpenaldehyde und -ketone wie z. B.; Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate; der cyclischen Terpenaldehyde und -ketone wie z. B.:; Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedernholzöl (Methylcedrylketon);
der cyclischen Alkohole wie z.B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan; der cyclischen und makrocyclischen Ketone wie z.B. ; 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. ; 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. ; 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B.; 2-tert-Butylcyclohexylacetat; 4-tert-Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Carbonsäuren wie z. B. ; Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. ; Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B.; Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxane; 4,4a,5,9b-Tetrahydroindeno[1,2-d]-m-dioxin; 4,4a,5,9b-Tetrahydro-2,4-dimethylindeno[1,2-d]-m-dioxin; der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Erfindungsgemäße Mischungen oder Verbindungen der Formel (I) können in flüssiger Form, unverdünnt oder mit einem Lösungmittel verdünnt eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat. Die genannten Lösungsmittel sind sehr gut geeignet, um die erfindungsgemäßen Mischungen oder die Verbindungen der Formel (I) zu lösen.

Des Weiteren können die erfindungsgemäßen Mischungen oder Verbindungen der Formel I an einem Trägerstoff adsorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulosebasierende Stoffe sein.

Die erfindungsgemäßen Mischungen oder Verbindungen der Formel I können auch mikroverkapselt, sprühgetrocknet, als Einschluß-Komplexe oder als Extrusions-Produkte vorliegen und in dieser Form dem zu parfümierenden Produkt hinzugefügt werden. Gegebenenfalls können die Eigenschaften dieser Darreichungsformen der erfindungsgemäßen Mischungen oder Verbindungen der Formel (I) durch sog "Coaten" mit geeigneten Materialien im Hinblick auf eine gezieltere Duftfreisetzung weiter optimiert werden, wozu vorzugsweise wachsartige Kunststoffe wie z.B. Polyvinylalkohol eingesetzt werden.

Die Mikroverkapselung kann beispielsweise durch das sogenannte Koazervationsverfahren mit Hilfe von Kapselmaterialien z.B. aus polyurethan-artigen Stoffen oder Weichgelatine, erfolgen. Sprühgetrocknete Produkte können beispielsweise durch Sprühtrocknung einer Emulsion, bzw. Dispersion hergestellt werden, wobei als Trägerstoffe modifizierte Stärken, Proteine, Dextrin und pflanzliche Gummen verwendet werden können. Einschluß-Komplexe können z.B. durch Mischen der erfindungsgemäßen Mischungen oder Verbindungen der Formel I mit Cyclodextrinen oder Harnstoffderivaten in einem geeigneten Lösungsmittel, z.B. Wasser, hergestellt werden. Extrusions-Produkte können durch Verschmelzen der erfindungsgemäßen Mischungen oder Verbindungen der Formel I mit einem geeigneten wachsartigen Stoff und durch Extrusion mit nachfolgender Erstarrung, ggf. in einem geeigneten Lösungsmittel, z.B. Isopropanol, erfolgen.
Konsumgüter sind beispielsweise Parfüms, Körperpflegeprodukte, wie Seifen, Duschgele, Shampoos, Badezusätze, Hautcremes, Körperlotionen und Deodorantien, und im Haushalt zu verwendende Reinigungs- oder Pflegeprodukte, wie Waschmittel, Wäscheweichspüler, Raumluftverbesserer und Reiniger. Weitere Beispiele für Konsumgüter werden nachfolgend genannt.
Die erfindungsgemäßen Mischungen oder die Verbindungen der Formel (I) sind sehr gut geeignet für den Einsatz in einer Vielzahl von Konsumgütern und können zum Beispiel in konzentrierter Form, in Lösungen oder in oben beschriebener modifizierter Form verwendet werden. Sie eignen sich für die Herstellung verschiedenster Konsumgüter, z.B. von Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und - lotionen, Gesichtscremes und -lotionen, Sonnenschutzcremes-und -lotionen, After-suncremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen. Der Vorteil des Einsatzes der erfindungsgemäßen Mischungen oder der Verbindungen der Formel (I) liegt darin, dass diese kompatibel mit einer Vielzahl von Inhaltstoffen der Konsumgüter sind, beispielsweise mit Tensiden, Säuren, Basen, Konservierungsmitteln Duftstoffen, d.h. dass die Funktionen einer Vielzahl von Inhaltsstoffen nicht beeinträchtigt wird. Ein weiterer Vorteil besteht in der sehr guten Bioabbaubarkeit der Verbindungen der Formel (I) und in der hohen Stabilität in Konsumgütern, die Wasser enthalten.
Ein weiterer Vorteil der Verbindungen der Formel (I) ist, dass diese in sehr geringen Konzentrationen eingesetzt werden können. Der geruchsverstärkende Effekt tritt in den erfindungsgemäßen Mischungen bereits bei einem Verhältnis aller Komponenten a zu allen Komponenten b von 1:10 bis 1:10000 ein. Beim Einsatz der erfindungsgemäßen Mischungen oder der Verbindungen der Formel (I) in Konsumgütern treten die genannten positiven Effekte bereits bei sehr geringen Konzentrationen auf, insbesondere wenn die Konzentration der mindestens einen Verbindung der Formel (I), bezogen auf das Gesamtgewicht des Konsumguts, 0,0001 bis 0,03 Gew.-% beträgt.

In einem weiteren Aspekt betrifft die vorliegende Erfindung daher die Verwendung der erfindungsgemäßen Mischungen oder der Verbindungen der Formel (I) zum Verstärken eines Geruchs, insbesondere von blumigen oder/und fruchtigen Noten.
Verbindungen der Formel (I) eignen sich auch zur Verwendung als Duftstoffe, insbesondere zur Erzeugung eines fruchtigen oder / und blumigen Dufts.
Die Verbindungen der Formel (I) lassen sich nach an sich bekannten Syntheseverfahren der organischen Chemie, wie zum Beispiel im Organikum, Deutscher Verlag der Wissenschaften, 18. Auflage, beschrieben, erhalten. Das als Edukt verwendete 1-(4-Methylcyclohexyl)-ethanol ist kommerziell erhältlich oder kann auch durch Hydrierung des entsprechenden Acetophenons erhalten werden.

Die nachfolgenden Beispiele sollen die Erfindung näher erläutern. Sofern nicht anders angegeben, beziehen sich alle Angaben auf das Gewicht. Die Reinigung der hergestellten Verbindungen erfolgte mittels Destillation oder präparativer Flüssigkeitschromatographie an Kieselgel.

### Beispiele

### Beispiel 1: 1-(4-Methylcyclohexyl)ethyl-propionat

142 g (1 Mol) 1-(4-Methylcyclohexyl)-ethanol, 88,8 g (1,2 Mol) Propionsäure und 1,2 g konzentrierte Schwefelsäure werden in einer 1L-Dreihalskolbenrührapparatur mit 400 ml Toluol 3 Stunden am Wasserabscheider erhitzt. Es wird zweimal mit 10%-iger Natriumcarbonat-Lösung und einmal mit 200 g Wasser gewaschen, getrocknet und eingeengt. Der verbleibende Rückstand wird über eine 10cm-Füllkörperkolonne destilliert. Man erhält 170 g Produkt mit einem GC-Gehalt (Summe der Isomeren) von 97,5% (Ausb.: 84% d. Th.).

Für analytische Untersuchungen wurden aus dem Rohprodukt die stereoisomeren Verbindungen der Formel (A) und (B) mittels Hochdruckflüssigkeitschromatographie an einer Phenomenex Luna C18 250*10mmm, 5µ mit dem Elutionsmittel Wasser/Methanol voneinander getrennt. Die Verbindung der Formel (A) wurde mit einer Reinheit von 99,3% und die Verbindung der Formel (B) mit einer Reinheit von 94,2% erhalten.
Verbindung der Formel (A): 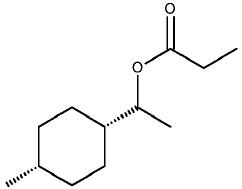 1H NMR (400 MHz, Chloroform-d) δ 4.89 (p, J = 7.7, 6.3 Hz, 1H), 2.31 (q, J = 7.5 Hz, 2H), 1.72 (tt, J =
   6.7, 4.1 Hz, 1H), 1.56 - 1.47 (m, 1H), 1.49 - 1.40 (m, 2H), 1.46 - 1.39 (m, 4H), 1.39 - 1.25 (m, 2H), 1.18
   (d, J = 6.3 Hz, 3H), 1.14 (t, J = 7.6 Hz, 3H), 0.91 (d, J = 7.0 Hz, 3H).
   13C NMR (101 MHz, CDCl3) δ = 174.20, 77.35, 77.03, 76.71, 72.75, 41.22, 30.88, 30.82, 29.04, 28.00, 24.37, 23.95, 19.24, 17.74, 9.30, -0.00.
Verbindung der Formel (B): 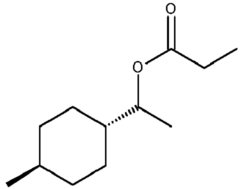 1H NMR (400 MHz, Chloroform-d) δ 4.73 (p, J = 6.4 Hz, 1H), 2.35 - 2.25 (m, 2H), 1.80 - 1.72 (m, 1H),
   1.76 - 1.67 (m, 2H), 1.65 (dt, J = 5.6, 3.2 Hz, 1H), 1.38 (dddp, J = 14.9, 12.8, 6.4, 3.2 Hz, 1H), 1.27
   (dddd, J = 16.3, 9.9, 5.7, 2.5 Hz, 1H), 1.18 - 1.14 (m, 3H), 1.13 (d, J = 7.6 Hz, 3H), 1.01 (dddd, J = 24.3,
   14.9, 12.3, 2.8 Hz, 2H), 0.97 - 0.87 (m, 2H), 0.87 (d, J = 6.5 Hz, 3H).
   13C NMR (101 MHz, CDCl3) δ = 174.18, 77.33, 77.02, 76.70, 74.40, 42.34, 34.81, 34.76, 32.64, 28.45, 28.39, 27.98, 22.59, 17.25, 9.29, 0.00.

### Beispiel 2: 1-(4-Methylcyclohexyl)ethyl-butyrat

29,8 g (0,21 Mol) 1-(4-Methylcyclohexyl)-ethanol, 103 g (0,65 Mol) Buttersäureanhydrid, 2,35 g (0,02 Mol) 4-Dimethylaminopyridin und 0,67 g (0,008 Mol) Pyridin werden in einer 250 ml-Dreihalskolbenrührapparatur 2 Stunden bei 120°C gerührt. Das Rohprodukt wird in eiskalte 5%-ige Salzsäure gegeben. Nach Extraktion mit
tert.-Butyl-methylether, Neutralwaschen und Trocknung wird am Rotationsverdampfer eingeengt. Anschließend wird über eine 10cm-Füllkörperkolonne destilliert. Man erhält 32,6 g Produkt mit einem GC-Gehalt (Summe der Isomeren) von 98,8% (Ausb.: 73% d. Th.).

### Beispiel 3: 1-(4-Methylcyclohexyl)ethyl-2-methylpropionat

50,0 g (0,35 Mol) 1-(4-Methylcyclohexyl)-ethanol, 37,2 g (0,42 Mol) 2-Methylpropionsäure und 0,5 g p-Toluolsulfonsäure werden in einer 250 ml-Dreihalskolbenrührapparatur mit 100 ml Toluol 4 Stunden am Wasserabscheider erhitzt. Nach Wäsche und Einengen am Rotationsverdampfer wird an einer 10cm-Füllkörperkolonne destilliert. Man erhält 30 g Produkt mit einem GC-Gehalt (Summe der Isomeren) von 98% (Ausb.: 40% d. Th.).

### Beispiel 4: 1-(4-Methylcyclohexyl)ethyl-2-hydroxypropionat

40,0 g (0,28 Mol) 1-(4-Methylcyclohexyl)-ethanol, 28,0 g (0,28 Mol) Milchsäure und 2,8 g p-Toluolsulfonsäure werden in einer 250 ml-Dreihalskolbenrührapparatur mit 100 ml Toluol 2 Stunden unter Rückfluss erhitzt. Das Rohprodukt wird mit Natriumhydrogencarbonat-Lösung und Wasser neutral gewaschen, getrocknet und am Rotationverdampfer eingeengt. Nach Destillation an einer 10cm-Füllkörperkolonne erhält man 33,8 g Produkt mit einem GC-Gehalt (Summe der Isomeren) von 97% (Ausb.: 55% d. Th.).

### Beispiel 5: 1-[1-(Ethoxymethoxy)ethyl]-4-methyl-cyclohexan

35,5 g (0,25 Mol) 1-(4-Methylcyclohexyl)-ethanol, 130 g (1,25 Mol) Formaldehyddiethylacetal, 1 g Lewatit K2641 und 100 g n-Hexan werden 10 Stunden bei 74°C gerührt. Während der Reaktionszeit werden 54 g einer Mischung aus Ethanol und n-Hexan abdestilliert. Nach Beendigung der Reaktion wird vom Lewatit abfiltriert. Das Filtrat wird eingeengt und anschließend über eine 10cm-Füllkörperkolonne destilliert. Man erhält 29,1 g Produkt mit einem GC-Gehalt (Summe der Isomeren) von 97% (Ausb.: 58% d. Th.).

### Beispiel 6: 1-Methyl-4-[1-(3-methylbut-2-enoxy)ethyl]cyclohexan

7,5 g Natriumhydrid, 250 g Toluol und 17,8 g (0,125 Mol) 1-(4-Methylcyclohexyl)-ethanol werden bei 100°C 1 Stunde (Beendigung der Wasserstoffentwicklung) erhitzt. Danach werden 30 g (0,25 Mol) Prenylchlorid zudosiert und weitere 2 Stunden bei 100°C gerührt. Nach Beendigung der Reaktion wird auf Eiswasser gegeben, tert.-Butyl-methylether zugefügt und die Phasen getrennt. Die organische Phase wird mit Kochsalzlösung und Wasser gewaschen, getrocknet und eingeengt. Das Produkt wird über eine 10cm-Füllkörperkolonne destilliert. Man erhält 20,7 g Produkt mit einem GC-Gehalt (Summe der Isomeren) von 95% (Ausb.: 75% d. Th.).

### Beispiel 7: Herstellung eines Parfümöles mit einer blumig-fruchtig-grün-holzigen Note.

Es werden die folgenden Riechstoffe in den angegebenen Mengen (Gewichtsteile) vermischt:

| | |
|---|---|
| ACETESSIGSAEUREETHYLESTER | 6 |
| CITROXAL 50% IN BB 10% DPG | 4 |
| HEXENOL CIS-3 | 1 |
| HEXENYLACETAT CIS-3 10% DPG | 7 |
| ALLYLAMYLGLYCOLAT | 1 |
| GALBEX TYPE BASE | 10 |
| LINALYLACETAT | 20 |
| CITRAL FF | 1,5 |
| ORANGENOEL BRAS. | 9 |
| ALDEHYD C14 SOG | 6 |
| MALTOL | 1,5 |
| LILIAL REPLACER BODYWASH 2 | 8 |
| HELIONAL | 15 |
| FLOROSA BM / PYRANOL | 40 |
| TETRAHYDROLI NALOOL | 20 |
| PHENYLETHYLALKOHOL | 70 |
| CITRONELLOL 950 | 9 |
| NEROL 900 | 15 |
| ROSAPHEN® | 20 |
| DAMASCONE DELTA 10% DPG | 8 |
| BENZYLACETAT | 12 |
| HEDION | 280 |
| JASMON CIS | 1 |
| VELOUTONE 10% DPG | 3 |
| AMYLSALICYLAT N | 4 |
| HEXENYLSALICYLAT CIS-3 | 12 |
| HEXYLSALICYLAT | 25 |
| ISORALDEIN 70 | 12 |
| CUMARIN 10% DPG | 5 |
| ISO E SUPER | 120 |
| TRIMOFIX O | 8 |
| VETIVAL® | 3 |
| VETIVERYLIA BASE | 5 |
| ISOBORNYLCYCLOHEXANOL | 40 |
| SANDRANOL® | 25 |
| EVERNYL | 3 |
| DIPROPYLENGLYCOL | 170 |
| Summe | 1000 |

In einem Duschgel wird mit der angegebenen Riechstoffkomposition nach Zugabe von 40 Gewichtsteilen 1-(4-Methylcyclohexyl)ethyl-propionat (Verbindung 1) und entsprechender Reduktion von 40 Gewichtsteilen des Lösungsmittels Dipropylenglycol die weiche blumige und fruchtige Note verstärkt (Boosting) und abgerundet. Außerdem wird ein citronellolartiger Rosengeruch erzeugt.

Die Parfümöle und eine Lösung der Verbindung 1 werden jeweils auf einen Reichstreifen aufgebracht und einer sensorischen Prüfung unterzogen. Dabei werden die Geruchsintensität der Blumigkeit und der Fruchtigkeit sensorisch auf einer Skala von 0 (keine Blumigkeit oder Fruchtigkeit) bis 10 (sehr starke Blumigkeit oder Fruchtigkeit) bewertet. Die Ergebnisse sind in der folgenden Tabelle dargestellt.

| Beispiel Nr. | Geruchsintensität der Blumigkeit | Geruchsintensität der Fruchtigkeit |
|---|---|---|
| 7 ohne Verbindung 1 | 4 | 2 |
| Lösung der Verbindung 1 * | 3 | 3 |
| 7 mit Verbindung 1 | 9 | 8 |

| | | |
|---|---|---|
| *40 Gewichtsteile der verbindung 1 gelöst in 960 Gewichtsteilen des praktisch geruchsfreien Lösungsmittels Dipropylenglycol | | |

### Beispiel 8: Herstellung eines Parfümöles mit einer blumig-fruchtig-rosigen-Holznote.

Es werden die folgenden Riechstoffe in den angegebenen Mengen (Gewichtsteile) vermischt:

| | |
|---|---|
| HEXENYLACETAT CIS,TRANS-3 10% DPG | 4 |
| VERTOCITRAL | 0,5 |
| MAGNOLAN | 20 |
| LINALYLACETAT | 25 |
| CITRONENOEL WINTER ITALIE | 10 |
| MANDARINENOEL ITAL. | 5 |
| RED BERRIES EXTRACT 10% DPG | 2 |
| CASSIS 345B TYPE BASE W/O MYRCENE | 8 |
| HELIONAL | 4 |
| FLOROSA | 15 |
| LINALOOL | 5 |
| PHENYLETHYLALKOHOL | 20 |
| CITRONELLOL 950 | 10 |
| GERANIOL 60 | 20 |
| DAMASCENON TOTAL 10% DPG | 5 |
| DAMASCON ALPHA 10% DPG | 1 |
| ROSE DE MAI-BASE | 8 |
| HEDION | 140 |
| HEXYLZIMTALDEHYD ALPHA | 20 |
| METHYLOCTINCARBONAT 1% DPG | 5 |
| IONON BETA | 8 |
| TABANON 1% DPG | 5 |
| CEDERNHOLZOEL VIRGINIA | 1,5 |
| AMBERWOOD® F | 5 |
| TIMBERSILK | 100 |
| VETIKOLACETAT® | 0,5 |
| AMBRA CORE | 3 |
| AMBROXIDE | 7 |
| AMBRETTOLIDE | 2 |
| GALAXOLID 50% IN IPM | 400 |
| INDOL FF 10% DPG | 1 |
| DIPROPYLENGLYCOL | 139,5 |
| | |
| Summe | 1000 |

Durch die Zugabe von 20 Gewichtsteilen 1-(4-Methylcyclohexyl)ethyl-propionat (aus Beispiel 1) und entsprechender Reduktion von 20 Gewichtsteilen des Lösungsmittels Dipropylenglycol wird in einem Eau de Toilette (EDT) eine interessante Pfingstrosennote hervorgerufen, die Natürlichkeit betont und die Fruchtigkeit verstärkt.

### Beispiel 9:

Die in Beispiel 8 angegebene Riechstoffkomposition einschließlich der hinzugefügten 20 Gewichtsteile 1-(4-Methylcyclohexyl)ethyl-propionat (aus Beispiel 1) wird mit einer Konzentration von 1 Gew.-% in ein Duschgel eingearbeitet und einem Stabilitätstest (4 Wochen Lagerung bei 40°C) unterzogen, wobei kein Verlust der Intensität der Pfingstrosennote, der Natürlichkeit und Fruchtigkeit eintritt.

### Beispiel 10: Herstellung eines Parfümöles mit einer blumig-fruchtig-grün-holzigen Note.

Es werden die folgenden Riechstoffe in den angegebenen Mengen (Gewichtsteile) vermischt:

| | |
|---|---|
| ACETESSIGSAEUREETHYLESTER | 7 |
| HEXENOL CIS-3 | 1 |
| HEXENYLACETAT CIS-3 10% DPG | 7 |
| ALLYLAMYLGLYCOLAT | 1 |
| GALBEX TYPE BASE | 10 |
| BERGAMOTTOEL BERGAPTENFREI | 20 |
| CITRONENOEL ITAL. | 20 |
| ORANGENOEL BRAS. | 12 |
| ALDEHYD C14 SOG | 6 |
| CASSIS 345B TYPE BASE | 3 |
| MALTOL | 1 |
| HELIONAL | 15 |
| FLOROSA BM / PYRANOL | 38 |
| HYDROXYCITRONELLAL GERANIUMOEL BOURBON 10% | 28 |
| DPG | 3 |
| PHENYLETHYLALKOHOL | 70 |
| DAMASCENON 10% DPG | 3 |
| BENZYLACETAT | 12 |
| HEDION | 155 |
| HEDION HC/30 | 185 |
| JASMON CIS | 1 |
| VELOUTONE 10% DPG | 2 |
| HEXENYLSALICYLAT CIS-3 | 33 |
| ISORALDEIN 95 | 17 |
| ETHYLVANILLIN 10% DPG | 0,5 |
| CUMARIN 10% DPG | 5 |
| ISO E SUPER NON DISCOLORING | 180 |
| TRIMOFIX O | 15 |
| ISOBORNYLCYCLOHEXANOL | 60 |
| SANDRANOL® | 40 |
| EVERNYL | 3 |
| AMBRETTOLIDE | 5 |
| EXALTENONE 942008 | 1,5 |
| GALAXOLID 50% IN IPM | 30 |
| DIPROPYLENGLYCOL | 10 |
| | |
| Summe | 1000 |

Die Zugabe von 10 Gewichtsteilen 1-(4-Methylcyclohexyl)ethyl-propionat (aus Beispiel 1) und entsprechende Reduktion von 10 Gewichtsteilen des Lösungsmittels Dipropylenglycol verleiht einem Eau de Toilette (EDT) mehr Lebendigkeit, Volumen, Blumigkeit und Fruchtigkeit.

## Patentansprüche

1. Mischung, enthaltend
a) mindestens einen Duftstoff der Formel (I) 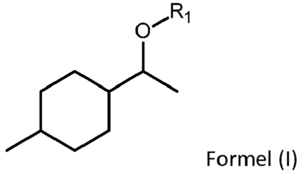 wobei
R1 = OC-R2, CH₂OR3, C1-C8-offenkettiger oder verzweigten aliphatischen Rest, gegebenenfalls substituiert und/oder ungesättigt,
mit R2 = einen offenkettigen oder verzweigten aliphatischen Rest, gegebenenfalls substituiert und/oder ungesättigt, mit 2-10 C-Atomen
mit R3 = einen offenkettigen oder verzweigten aliphatischen Rest, gegebenenfalls substituiert und/oder ungesättigt, mit 1-8 C-Atomen
bedeuten.
b) mindestens einen Duftstoff, verschieden von den Duftstoffen der Komponente a,
**dadurch gekennzeichnet, dass** das Gewichtsverhältnis aller Komponenten a zu allen Komponenten b von 1:10 bis 1:10000 beträgt.

2. Mischung nach Anspruch 1, **dadurch gekennzeichnet, dass** R1 ein Propanoyl-, Butanoyl-, 2-Methylpropanoyl-, 2-Hydroxypropanoyl-, Methoxyethyl- oder 2-Methylbut-2-enyl- Rest ist.

3. Mischung nach Anspruch 1, **dadurch gekennzeichnet, dass** der Duftstoff (Komponente a) ausgewählt ist aus 1-(4-Methylcyclo-hexyl)ethyl-propionat, 1-(4-Methylcyclo-hexyl)ethyl-butyrat, 1-(4-Methylcyclo-hexyl)ethyl-isobutyrat, 1-(4-Methylcyclo-hexyl)ethyl-2-hydroxy-propionat, 1-[1-Ethoxy-methoxy]ethyl]-4-methylcyclohexan und 1-Methyl-4-[1-(3-Methylbut-2-enoxy)-ethyl]-cyclohexan.

4. Duftstoffmischung enthaltend mindestens zwei Duftstoffe der Formel (I).

5. Verbindung der Formel

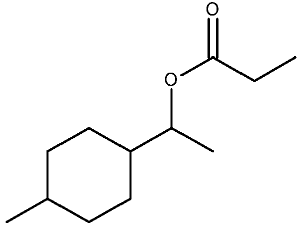

6. Verbindung der Formel

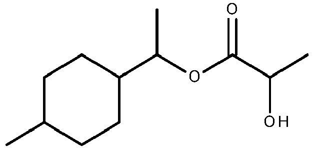

7. Verbindung der Formel

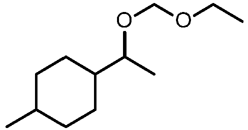

8. Verbindung der Formel

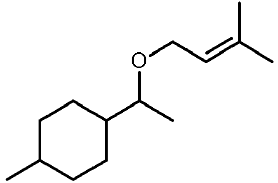

9. Konsumgut enthaltend eine Mischung nach Anspruch 1 oder mindestens eine Verbindung nach den Ansprüchen 5 bis 8.

10. Konsumgut nach Anspruch 9 enthaltend Wasser.

11. Konsumgut nach Anspruch 10, **dadurch gekennzeichnet, dass** der Anteil des Wassers in dem Konsumgut 5,0 bis 99,0 Gew.-% beträgt.

12. Verwendung einer Verbindung nach einem der Ansprüche 1, 2, 3, 5, 6, 7 oder als Duftstoff, zum Verstärken positiver Geruchseindrücke oder / und zum Verstärken von blumigen und fruchtigen Geruchseindrücken.

## Claims

1. Mixture comprising
a) at least one fragrance of the formula (I) 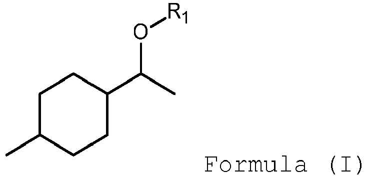 where
R1 = OC-R2, CH₂OR3, C1-C8 open-chain or branched aliphatic radical, optionally substituted and/or unsaturated,
with R2 = an open-chain or branched aliphatic radical, optionally substituted and/or unsaturated, having 2-10 C atoms,
with R3 = an open-chain or branched aliphatic radical, optionally substituted and/or unsaturated, having 1-8 C atoms,
b) at least one fragrance, different from the fragrances of component a,
**characterized in that** the weight ratio of all components a to all components b is from 1:10 to 1:10000.

2. Mixture according to Claim 1, **characterized in that** R1 is a propanoyl, butanoyl, 2-methylpropanoyl, 2-hydroxypropanoyl, methoxyethyl or 2-methylbut-2-enyl radical.

3. Mixture according to Claim 1, **characterized in that** the fragrance (component a) is selected from 1-(4-methylcyclo-hexyl)ethyl propionate, 1-(4-methylcyclo-hexyl)ethyl butyrate, 1-(4-methylcyclo-hexyl)ethyl isobutyrate, 1-(4-methylcyclo-hexyl)ethyl 2-hydroxypropionate, 1-[1-ethoxy-methoxy]ethyl]-4-methyl cyclohexane and 1-methyl-4-[1-(3-methylbut-2-enoxy)ethyl]cyclohexane.

4. Fragrance mixture comprising at least two fragrances of the formula (I).

5. Compound of the formula

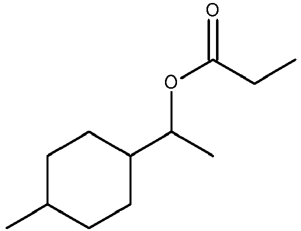

6. Compound of the formula

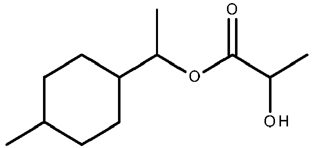

7. Compound of the formula

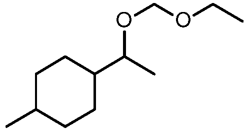

8. Compound of the formula

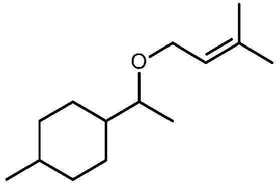

9. Consumer product comprising a mixture according to Claim 1 or at least one compound according to Claims 5 to 8.

10. Consumer product according to Claim 9, comprising water.

11. Consumer product according to Claim 10, **characterized in that** the fraction of water in the consumer product is 5.0 to 99.0 wt%.

12. Use of a compound according to any of Claims 1, 2, 3, 5, 6, 7 or as fragrance, for boosting positive odour impressions and/or for boosting flowery and fruity odour impressions.

## Revendications

1. Mélange contenant
a) au moins un parfum de formule (I), 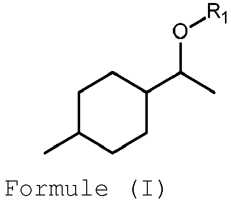 dans laquelle
R1 = OC-R2, CH₂OR3, un radical aliphatique à chaîne ouverte ou ramifiée en C1-C8, le cas échéant substitué et/ou insaturé,
où R2 = un radical aliphatique à chaîne ouverte ou ramifiée, le cas échéant substitué et/ou insaturé, comprenant 2-10 atomes de carbone,
où R3 = un radical aliphatique à chaîne ouverte ou ramifiée, le cas échéant substitué et/ou insaturé, comprenant 1-8 atomes de carbone,
b) au moins un parfum, différent des parfums du composant a,
**caractérisé en ce que** le rapport pondéral de tous les composants a à tous les composants b est de 1:10 à 1:10000.

2. Mélange selon la revendication 1, **caractérisé en ce que** R1 représente un radical propanoyle, butanoyle, 2-méthylpropanoyle, 2-hydroxypropanoyle, méthoxyéthyle ou 2-méthylbut-2-ényle.

3. Mélange selon la revendication 1, **caractérisé en ce que** le parfum (composant a) est choisi parmi le propionate de 1-(4-méthylcyclohexyl)éthyle, le butyrate de 1-(4-méthylcyclohexyl)éthyle, l'isobutyrate de 1-(4-méthylcyclohexyl)éthyle, le 2-hydroxypropionate de 1-(4-méthylcyclohexyl)éthyle, le 1-[1-éthoxyméthoxy]éthyl]-4-méthylcyclohexane et le 1-méthyl-4-[1-(3-méthylbut-2-énoxy)-éthyl]-cyclohexane.

4. Mélange de parfums contenant au moins deux parfums de formule (I).

5. Composé de formule

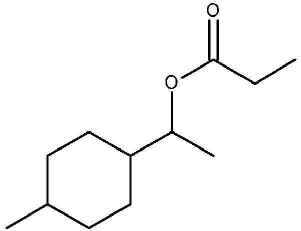

6. Composé de formule

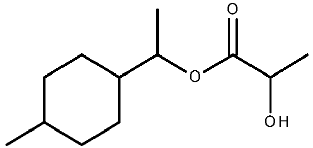

7. Composé de formule

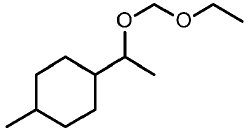

8. Composé de formule

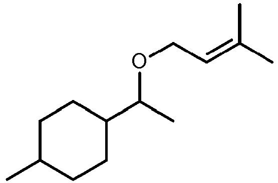

9. Produit de consommation contenant un mélange selon la revendication 1 ou au moins un composé selon les revendications 5 à 8.

10. Produit de consommation selon la revendication 9 contenant de l'eau.

11. Produit de consommation selon la revendication 10, **caractérisé en ce que** la proportion d'eau dans le produit de consommation est de 5,0 à 99,0% en poids.

12. Utilisation d'un composé selon l'une quelconque des revendications 1, 2, 3, 5, 6, 7 ou comme parfum, pour le renforcement d'impressions olfactives positives et/ou pour le renforcement d'impressions olfactives florales et fruitées.
